# FASCICULE DE BREVET EUROPEEN

(11) **EP 4 343 309 B1**
(45) Date de publication et mention de la délivrance du brevet: **05.08.2026**
(21) Numéro de dépôt: 23198815.5
(22) Date de dépôt: 21.09.2023
(51) Int. Cl.: G01N 21/3563, G01N 21/359, G01N 21/64, G01N 33/02, G01N 21/63, G01N 21/55, G01N 21/84

(54) **TRI OPTIQUE DE FRUITS NON-CÉRÉALIERS PAR COMBINAISON DE RAYONNEMENTS ÉLECTROMAGNÉTIQUES**
OPTISCHE SORTIERUNG VON NICHTZEREBRATEN FRÜCHTEN DURCH KOMBINATION ELEKTROMAGNETISCHER STRAHLUNG
OPTICAL SORTING OF NON-CEREAL FRUITS BY COMBINING ELECTROMAGNETIC RADIATION

(30) Priorité: 21.09.2022 FR 2209553
(43) Date de publication de la demande: 27.03.2024
(73) Titulaire: Bucher Vaslin, 49290 Chalonnes sur Loire (FR)
(72) Inventeur: MARCHAND, Stéphane, 49290 CHALONNES-SUR-LOIRE (FR); POINEL, Julien, 49290 CHALONNES-SUR-LOIRE (FR)
(74) Mandataire: Ipsilon

(56) Documents cités:
- EP-A1- 3 379 246
- WO-A2-2007/017732
- US-A1- 2005 098 713
- ARIANA D ET AL: "Integrating multispectral reflectance and fluorescence imaging for defect detection on apples", COMPUTERS AND ELECTRONICS IN AGRICULTURE, ELSEVIER, AMSTERDAM, NL, vol. 50, no. 2, 1 February 2006 (2006-02-01), pages 148 - 161, XP024956988, ISSN: 0168-1699, [retrieved on 20060201], DOI: 10.1016/J.COMPAG.2005.10.002
- W. MEULEBROECK ET AL: "<title>Optical detection techniques for laser sorting machines</title>", PROCEEDINGS OF SPIE, vol. 6189, 21 April 2006 (2006-04-21), US, pages 61891F, XP055267017, ISBN: 978-1-5106-1533-5, DOI: 10.1117/12.660896

## Description

L'invention se rapporte à un procédé non destructif de détermination de la maturité et de la qualité d'un fruit non-céréalier, un procédé de tri de fruits non-céréaliers et un appareil de tri de tels fruits non-céréaliers mettant en œuvre ces procédés.

L'industrialisation de techniques de tri à haut débit de produits agro-alimentaires issus de cultures est essentielle pour une production de masse, en particulier pour trier les produits en fonction de leur maturité.

Or, il est connu que la maturité des produits peut être évaluée par la détermination de composés chromophores contenus dans les cellules des végétaux. La maturité visée peut concerner essentiellement la maturité phénolique. Ce type de système offre la possibilité de pouvoir être utilisé en ligne et s'insérer dans une ligne de production.

Il est connu par exemple de JPH11621A et KR20030088706, des procédés de tri de grains de riz par une exposition à des rayonnements électromagnétiques et à l'analyse des signaux.

De la même façon, dans EP0898701 il est rapporté un procédé pour déterminer la maturité et la qualité de graines par irradiation électromagnétique.

Ainsi, ces procédés sont particulièrement bien adaptés aux fruits/produits céréaliers, ayant typiquement un nombre restreint de marqueurs de maturité.

L'un des avantages de ces procédés est en outre la rapidité de l'étape de sélection de graines ou de grains de riz, ce qui les rend particulièrement attrayants pour leur mise en application dans des lignes de production à fort débit. En outre, la fluorescence de la chlorophylle est un facteur déterminant dans les procédés ainsi décrits.

Concernant des fruits non-céréaliers, il a été rapporté par G. Agati et al. ("Nondestructive evaluation of anthocyanins in olive (Olea europaea) fruits by in situ chlorophyll fluorescence spectroscopy"; J Agric Food Chem. 2005, (5), 1354-63) que les anthocyanes dans les fruits d'olive (Olea europaea L.) à différents degrés de pigmentations ont été évaluées de manière non destructive en mesurant la fluorescence de la chlorophylle. Ceci a été réalisé par une méthode basée sur la comparaison des spectres d'excitation de la chlorophylle de différentes olives présentant des pigmentation différentes, variant du vert au vert-rouge, rouge-violet et violet. Toutefois, cette technique est limitée à des fruits dits « en basse » ou « moyenne maturité ». Les irradiations avec des lumières vertes et rouges ne permettent pas d'apprécier l'échelle totale de la maturité.

Or, dans WO 00/02036, il est décrit l'utilisation de la fluorescence pour déterminer la présence ou la teneur en chlorophylle d'un végétal, en particulier dans des baies. Toutefois, pour les baies les plus mûres, le niveau de chlorophylle peut être très faible. Ce faible signal de fluorescence de la chlorophylle peut même être insuffisant pour être détecté, ce qui peut occasionner alors de erreurs de tri et une baisse qualitative du produit trié. La technique est donc perfectible. De plus, dans WO 00/02036, les puissances décrites ne sont pas suffisantes surtout dans le vert : la technologie décrite semble donc limitée au criblage d'une population dite à maturité.

Le document EP3379246A1 divulgue un procédé non destructif de détermination de la maturité et de la qualité d'un fruit non-céréalier pouvant comprendre deux étapes d'expositions dudit fruit à au moins deux rayonnements électromagnétiques différents et de mesures de leurs signaux retour. Toutefois, le document EP3379246A1 présente des limites pratiques de mise en œuvre, notamment quant à la qualité des signaux obtenus, ce qui a un impact direct sur la qualité des fruits isolés.

Le but de l'invention est donc de pallier les inconvénients de l'art antérieur et vise ainsi en particulier à surmonter les difficultés de tri de fruits non-céréaliers à l'échelle industrielle.

En outre, de manière générale, le signal de fluorescence issue de la chlorophylle est dépendant la puissance de l'excitation et du niveau de chlorophylle. Des faibles teneurs de chlorophylle nécessitent des puissances d'éclairage qui présente une difficulté technique (c'est-à-dire une limite technologique) et économique.

### RESUME DE L'INVENTION

Pour ce faire, un premier aspect de l'invention se rapporte ainsi, dans son acceptation la plus large, à un procédé non destructif de détermination de la maturité et de la qualité d'un fruit non-céréalier selon la revendication 1 et comprenant au moins deux étapes d'expositions dudit fruit à au moins deux rayonnements électromagnétiques différents et de mesures de leurs signaux retours, dans lequel :
- l'une des étapes d'expositions dudit fruit comprend la fourniture d'au moins une longueur d'onde susceptible de provoquer un signal de fluorescence de chlorophylle et une mesure, le cas échéant, dudit signal de fluorescence de chlorophylle ; et
- l'une des étapes d'exposition dudit fruit à au moins un rayonnement électromagnétique comprend la fourniture d'au moins une longueur d'onde choisie, susceptible de générer un signal de réflexion sur ledit fruit, et une mesure, le cas échéant, dudit signal de réflexion, caractérisé, en particulier, en ce que ladite au moins une longueur d'onde susceptible de provoquer une fluorescence de la chlorophylle est comprise entre 590 nm et 660 nm.

En effet, d'autres marqueurs de maturité que la chlorophylle sont connus (telles que les anthocyanes), qui contribuent à produire les couleurs caractéristiques des fruits. Il s'opère ainsi une contribution mutuelle des différents marqueurs de maturité qui se traduit par des variations de taux de ces différents marqueurs dans les fruits. La présente invention utilise ce concept pour aider le processus d'évaluation de maturité des fruits non-céréaliers, et ainsi aider à augmenter la précision du tri industriel de tels fruits.

L'un des principes de la présente invention est ainsi de pouvoir corréler un signal issu d'une réflexion électromagnétique choisie en fonction du fruit trié avec un signal de fluorescence de chlorophylle. Les signaux recueillis sont ainsi complémentaires en ce qu'ils fournissent des informations différentes utiles au tri. Ainsi, grâce au procédé selon la présente invention, la sélection devient plus précise et le tri de meilleure qualité.

En particulier, en ce qui concerne les gammes de valeurs de longueur d'onde susceptible de provoquer une fluorescence de la chlorophylle est comprise entre 590 nm et 660 nm, celles-ci permettent une meilleure spécificité des irradiations et donc une meilleure certitude des résultats. L'un des avantages de l'invention est de pouvoir distinguer deux signaux différents mais complémentaires quant aux caractéristiques d'un fruit particulier, spécifiquement le raisin. En effet, il a été découvert, en particulier pour le raisin, que les anthocyanes ne masquent pas la fluorescence de la chlorophylle aux gammes de valeurs de longueur d'onde émises lorsque la chlorophylle fluoresce par l'excitation à longueur d'onde 590 nm et 660 nm. Ainsi, lorsque la longueur d'onde d'excitation est comprise entre 590 et 660 nm, le signal émis (fluorescence de la chlorophylle) est complémentaire aux signaux de réflexion des anthocyanes présents et l'ensemble des données collectées permet de mieux caractériser qualitativement le fruit au regard des techniques de l'état de l'art connu.

L'objet de la présente invention concerne également un appareil de tri de fruits non-céréaliers selon la revendication 8, l'appareil comprenant :
- un dispositif d'alimentation de fruits non-céréaliers,
- optionnellement au moins un moyen d'écarter des objets qui ne sont pas des fruits ;
- au moins un système de rayonnement configuré pour générer au moins un rayonnement électromagnétique ;
- au moins une zone de détection configurée pour mesurer et optionnellement analyser les signaux retours issus dudit au moins un système de rayonnement ;
- un système de séparation desdits fruits activable en fonction des signaux retours, caractérisé en ce que :
   - ledit au moins un système de rayonnement est configuré de manière à exposer lesdits fruits à au moins deux rayonnements électromagnétiques différents ;
   - l'un desdits au moins deux rayonnements électromagnétiques comprend au moins une longueur d'onde susceptible de provoquer une fluorescence de chlorophylle ;
   - l'un desdits au moins deux rayonnements électromagnétiques comprend au moins une longueur d'onde choisie pour générer une réflexion sur ledit fruit, telle que 750 nm ;
   - ledit au moins un système de rayonnements et la zone de détection sont disposés de manière à :
      - ce qu'au moins deux signaux retours générés par ledit au moins un système de rayonnement sont captés sur une même zone de détection, ou
      - être d'un même côté des fruits (dans le cas contraire, il serait possible que le support du fruit soit transparent par exemple)
caractérisé en particulier, en ce que ladite au moins une longueur d'onde susceptible de provoquer une fluorescence de la chlorophylle est comprise entre 590 nm et 660 nm, et en ce que les mesures du signal de fluorescence de la chlorophylle et du signal de réflexion sont effectuées par un même capteur.

Ainsi, l'appareil selon la présente invention permet une amélioration du tri de fruits non-céréaliers grâce à un signal complémentaire au signal obtenu par l'excitation fluorescente de la chlorophylle. Ce signal complémentaire est calibré spécifiquement au type de fruit que l'on souhaite trier. En outre, contrairement à des appareils permettant de trier certaines céréales tel que le riz, les systèmes de rayonnements et les zones de détections sont placés du même côté du fruit. En effet, dans le riz est partiellement translucide ce qui impose une configuration différente des systèmes de détection et d'émission par rapport à d'autres fruits non-céréaliers.

La présente invention permet en outre d'identifier les objets qui ne sont pas des fruits : si aucun signal de fluorescence de chlorophylle n'est capté et que le signal de réflexion n'est pas caractéristique du fruit analysé, il s'agit donc d'un corps étranger qui peut être retiré. D'autre manières de distinguer les objets autres que des fruits peuvent être utilisés (systèmes de mesure basés sur le poids par exemple).

L'objet de la présente invention concerne en outre un procédé de tri de fruits non-céréaliers selon la revendication 9, le procédé comprenant les étapes suivantes :
- amener individuellement chaque fruit non-céréaliers à au moins une zone d'exposition,
- exposer ledit fruit dans ladite au moins une zone d'exposition à au moins deux rayonnements électromagnétiques différents,
- réceptionner et analyser les signaux retours de l'étape d'exposition,
- séparer lesdits fruits en différentes classes en fonction de leur signaux individuels,
dans lequel :
- l'un des au moins deux rayonnements électromagnétiques différents comprend au moins une longueur d'onde susceptible de provoquer une fluorescence de chlorophylle comme signal retour ;
- l'un des au moins deux rayonnements électromagnétiques différents comprend au moins une longueur d'onde choisie pour générer une réflexion sur ledit fruit comme signal retour ; et
- les valeurs qui définissent les classes sont préétablies ou choisies en fonction d'une répartition des signaux de réflexion et de fluorescence de chlorophylle émis par un échantillon de fruits non-céréaliers possédant des propriétés connues,
caractérisé en particulier, en ce que ladite au moins une longueur d'onde susceptible de provoquer une fluorescence de la chlorophylle est comprise entre 590 nm et 660 nm, et en ce que les mesures du signal de fluorescence de la chlorophylle et du signal de réflexion sont effectuées par un même capteur.

Ainsi, ce procédé permet d'utiliser, si on le souhaite, une valeur seuil prédéfinie, ou de choisir ce seuil de tri des fruits non-céréaliers grâce à une étude préliminaire d'un échantillon de la récolte que l'on souhaite trier. La présente invention permet donc de maintenir si on le souhaite un niveau constant, que l'on pourrait qualifier de « normé », de la qualité des fruits triés, ou au contraire d'adapter le tri à la réalité de la récolte et ainsi d'ajuster par exemple le rapport entre un rendement de production suffisant et la qualité des fruits récoltés.

L'invention (procédés et appareil de tri) est donc particulièrement bien adaptée pour le tri de fruits tels que des raisins dans le cadre de la production de produit dérivés de fruits non-céréaliers telles que des boissons comme du vin, des jus de fruits, des coulis, etc., des produits laitiers comprenant des fruits ou des parties de fruits, comme des yaourts aux fruits, ou encore des desserts aux fruits telles que des tartes, gâteaux, mousses etc. qui sont particulièrement sensibles à la qualité des fruits utilisés.

### DEFINITIONS

Par « fruit non-céréalier », il est compris dans le cadre de la présente invention, un fruit qui n'est pas un fruit céréalier. Par fruit « céréalier », il est compris dans le cadre de la présente invention, la définition botanique d'un fruit, c'est-à-dire un grain, ou encore une semence, d'une céréale. Les céréales sont des plantes monocotylédones de la famille des graminées, sauvages ou cultivées, qui produisent des grains comestibles, principalement utilisées en alimentation humaine, souvent moulues sous forme de farine raffinée ou plus ou moins complète. Des exemples, de céréales sont le maïs, le blé, le riz, l'orge, le sorgho, l'avoine, le seigle, le mil, le triticale.

Par « zone de détection », il est compris dans le cadre de la présente invention une surface sensible à au moins un rayonnement électromagnétique (i.e., une lumière) et configuré pour transformer un signal lumineux en un signal utilisable, tel qu'un signal électrique. Typiquement, une telle zone de détection est une caméra, telle qu'une caméra numérique comme une caméra CCD, CMOS. La caméra peut être matricielle ou linéaire. La zone de détection peut en outre être pourvue d'un ou plusieurs filtres lumineux pour éliminer les signaux lumineux n'ayant aucun intérêt dans le cadre de la présente invention et ainsi améliorer le rapport signal / bruit de fond.

Par « signal retour », il est compris dans le contexte de la présente invention le signal provenant du fruit vers un capteur. Ce signal retour peut être un signal de fluorescence de la chlorophylle ou un signal de réflexion.

Par « signal de fluorescence », il est compris dans le contexte de la présente invention le signal issu de l'excitation de la chlorophylle.

Par « signal de réflexion », il est compris dans le contexte de la présente invention le signal lumineux qui n'est pas absorbé par le fruit non-céréalier et qui est donc réfléchi.

Par « même côté d'un fruit », il est compris dans le contexte de la présente invention une face du fruit vue d'un seul point extérieur du fruit.

### DESCRIPTION DETAILLEE

### • Procédé de détermination de la maturité et de la qualité d'un fruit :

La présente invention concerne en particulier un procédé non destructif de détermination de la maturité et de la qualité d'un fruit non-céréalier, tel que du raisin, comprenant :
- une première étape d'exposition dudit fruit à au moins un rayonnement électromagnétique et de mesure du signal retour,
- une seconde étape d'exposition dudit fruit à au moins un rayonnement électromagnétique différent de celui de la première étape et de mesure du signal retour,
dans lequel :
- l'une des étapes d'expositions dudit fruit comprend la fourniture d'au moins une longueur d'onde susceptible de provoquer un signal de fluorescence de chlorophylle et une mesure, le cas échéant, dudit signal de fluorescence de chlorophylle ; et
- l'une des étapes d'exposition dudit fruit à au moins un rayonnement électromagnétique comprend la fourniture d'au moins une longueur d'onde choisie susceptible de générer un signal de réflexion sur ledit fruit, et une mesure, le cas échéant, dudit signal de réflexion.

Selon l'invention, le procédé selon la présente invention se caractérise en ce que les mesures du signal de fluorescence de la chlorophylle et du signal de réflexion sont effectuées par un même capteur.

Il y a plusieurs avantages à l'utilisation d'un seul capteur pour la collecte des deux signaux retours selon l'invention. Par exemple, un seul capteur permet de limiter le coût financier du matériel. Un seul capteur permet de limiter la maintenance sur le matériel. Un seul capteur permet une compacité accrue du matériel. Un seul capteur implique que les signaux de fluorescence de chlorophylle et de réflexion proviennent d'une même face du fruit analysé, ce qui permet de mieux déduire une qualité générale du fruit grâce à une corrélation plus précise entre les deux signaux.

Ainsi, de manière avantageuse, le procédé selon la présente invention peut être caractérisé en ce que le capteur est une caméra optionnellement pourvue d'un filtre passe-bande, par exemple un filtre passe-bande calibré à 750 nm.

Ainsi, dans un mode de réalisation particulier, le procédé selon la présente invention peut être caractérisé en ce que d'une part, la fourniture d'au moins une longueur d'onde susceptible de provoquer un signal de fluorescence de chlorophylle, et d'autre part la fourniture d'au moins une longueur d'onde choisie susceptible de générer un signal de réflexion sur ledit fruit, sont séquencés (dans le temps de manière distincte) et/ou synchronisés, avec optionnellement au moins un temps de pause pour permettre la mesure du bruit de fond.

Dans un mode de réalisation particulier, les systèmes de rayonnement comprennent une ou plusieurs sources à base de diodes électroluminescentes (LED).

De manière préférée, le procédé selon la présente invention peut être caractérisé en ce que ladite au moins une longueur d'onde choisie pour générer une réflexion sur ledit fruit est choisie avec une longueur d'onde significativement supérieure à la longueur d'onde d'excitation de la chlorophylle.

Par « significativement supérieure à la longueur d'onde d'excitation de la chlorophylle », il est compris dans le contexte de la présente invention une longueur d'onde qui ne génère pas d'excitation de chlorophylle susceptible d'être détectée (en particulier en intensité de signal) par le capteur employé.

De manière plus préférée, le procédé selon la présente invention peut être caractérisé en ce que la longueur d'onde significativement supérieure à la longueur d'onde d'excitation de la chlorophylle est comprise entre 700 nm et 900 nm, préférentiellement de 750 nm plus ou moins 10 nm.

Par exemple, le procédé selon la présente invention peut être caractérisé en ce que la longueur d'onde significativement supérieure à la longueur d'onde d'excitation de la chlorophylle est comprise entre 710 nm et 850 nm, entre 720 nm et 800 nm, entre 730 nm et 780 nm ou encore entre 735 nm et 765 nm.

De manière plus préférée, le procédé selon la présente invention peut être caractérisé en ce que ladite au moins une longueur d'onde susceptible de provoquer une fluorescence de la chlorophylle est comprise entre 550 nm et 680 nm, préférentiellement de 590 nm et 660, plus préférentiellement de 625 nm plus ou moins 10 nm.

Par exemple, le procédé selon la présente invention peut être caractérisé en ce que ladite au moins une longueur d'onde susceptible de provoquer une fluorescence de la chlorophylle est comprise entre 590 nm et 660 nm, entre 600 nm et 650 nm ou encore entre 610 nm et 640 nm, en particulier de 625 nm plus ou moins 10 nm

Dans un mode de réalisation particulier, le procédé selon la présente invention peut être caractérisé en ce que la longueur d'onde significativement supérieure à la longueur d'onde d'excitation de la chlorophylle est comprise entre 700 nm et 900 nm, préférentiellement de 750 nm plus ou moins 10 nm, et en ce que ladite au moins une longueur d'onde susceptible de provoquer une fluorescence de la chlorophylle est comprise entre 550 nm et 680 nm, préférentiellement de 625 nm plus ou moins 10 nm.

L'avantage est de pouvoir distinguer deux signaux différents mais complémentaires quant aux caractéristiques d'un fruit particulier.

Par exemple, un fruit comme le raisin, comprend au moins deux marqueurs de maturité : de la chlorophylle et des anthocyanes. La chlorophylle peut être excitée avec une onde électromagnétique de longueur d'onde comprise entre 550 et 680 nm, et la fluorescence ainsi générée peut être comprise entre 720 et 770 nm, telle que 750 nm plus ou moins 10 nm. Lorsque la longueur d'onde d'excitation est comprise entre 590 et 660 nm, le signal émis (fluorescence de la chlorophylle) est complémentaire à ceux des anthocyanes présents et permet de caractériser qualitativement le fruit.

Ainsi, l'une des caractéristiques distinctives de la présente invention est que la chlorophylle est détectée par fluorescence et le signal de réflexion permet d'avoir des informations complémentaires (e.g. présence d'un objet et forme de l'objet irradié).

Pour réaliser la mesure d'un fruit comme le raisin, le fruit peut subir une première excitation séquentielle rouge (par exemple 625 nm) et une émission d'un second flux de lumière, par exemple 750 nm. Le premier signal retour est donc caractéristique de la fluorescence de la chlorophylle (par exemple environ 750 nm) et le second signal retour issu de la réflexion (par exemple environ 750 nm) sur le fruit de la deuxième émission à 750 nm, caractéristique de la présence des anthocyanes (second marqueur de maturité).

Dans un mode de réalisation particulier et avantageux, le support qui permet d'amener le fruit dans la zone d'analyse (là où il va être irradié), n'interagit pas ou peu avec les ondes électromagnétiques aux longueurs d'ondes choisies. Ainsi, les signaux recueillis ne sont pas issus du support. Par exemple le support peut comprendre une partie vitrée, transparente pour éviter de polluer le signal.

Ainsi, les puissances d'irradiation (tel que dans l'infra-rouge) pour que le support ne sature pas la caméra, sont adaptés spécifiquement aux fruits analysés. Le signale retour des fruits n'est pas noyé dans une masse de signal qui proviendrait du support.

De plus, dans le contexte de la présente invention, la teneur en chlorophylle résiduelle d'un fruit comme le raisin est mesurée en évitant l'effet masquant des anthocyanes après excitation avec une longueur d'onde appropriée (par exemple 750 nm).

Dans ce cas de figure particulier dans lequel les marqueurs de maturité sont la chlorophylle et des anthocyanes, il peut être particulièrement intéressant d'utiliser une seule zone de détection (par exemple une seule caméra) étant donné que les longueurs d'ondes des signaux retour sont proches, voire identiques (par exemple environ 750 nm)

Dans le cas de figure d'une utilisation d'une seule zone de détection, telle qu'une caméra, il est nécessaire de procéder à des excitations séquentielles par exemple par l'utilisation d'une caméra filtrée passe bande pour permettre de mesurer la fluorescence sur une longueur d'onde en synchronisation avec chaque excitation, et mesurer le signal retour issu de la réflexion sur une longueur d'onde en synchronisation avec chaque excitation.

Il peut être avantageux selon le procédé, que les ondes d'excitations et d'émissions soient filtrées passe haut (fréquence) pour supprimer les longueurs d'ondes proches de la longueur d'onde de fluorescence.

Il peut être avantageux selon le procédé, que le système s'étalonne en permanence en incluant une séquence de mesure sans excitation pour discriminer le bruit de fond.

Ainsi, le procédé selon la présente invention peut être appliqué à tout type de fruit non-céréalier comprenant de la chlorophylle et un second marqueur de maturité dont le signal retour obtenu par réflexion ou le signal retour obtenu par excitation diffère des longueurs d'ondes d'excitation et de fluorescence de la chlorophylle. Ceci dépend donc directement de la nature du fruit non céréalier.

De manière préférée, le procédé selon la présente invention peut être caractérisé en ce que le fruit non-céréalier comprend de la chlorophylle et au moins un second marqueur de maturité utile pour la réflexion. En effet, en absence de chlorophylle le second marqueur réfléchira la lumière (ondes électromagnétiques) auquel le fruit est soumis.

Ainsi, de manière préférée, le procédé selon la présente invention peut être caractérisé en ce que le fruit non-céréalier contient au moins un pigment (ou marqueur de maturité) choisi parmi le lycopène, le bêtacarotène, l'anthocyane et la lutéine, préférentiellement l'anthocyane et/ou le lycopène.

De manière préférée, le procédé selon la présente invention peut être caractérisé en ce que le fruit non-céréalier est un fruit charnu.

Un fruit charnu est un fruit dont le mésocarpe a la consistance d'une pulpe, telles que les baies ou des drupes.

De manière préférée, le procédé selon la présente invention peut être caractérisé en ce que le fruit non-céréalier est choisi parmi une tomate, un raisin, une fraise, une framboise, une groseille, une cerise, une mûre, une airelle rouge, un cassis, une baie d'aronia, une canneberge, un sureau noir et une myrtille, préférentiellement un raisin tel qu'un raisin rouge.

De manière préférée, le procédé selon la présente invention peut être caractérisé en ce que le fruit non-céréalier est un fruit rouge.

Dans le contexte de la présente invention, un fruit rouge est une baie telle qu'un raisin, une fraise, une framboise, une groseille, une cerise, une mûre, une airelle rouge, un cassis, une baie d'aronia, une canneberge, un sureau noir ou une myrtille, préférentiellement un raisin tel qu'un raisin rouge.

En outre, il peut être avantageux d'ajouter au procédé selon la présente invention une technique complémentaire connue de tri basé sur la couleur du fruit non-céréalier.

### • Procédé de tri de fruits non-céréaliers :

L'objet selon la présente invention comprend ainsi un procédé de tri de fruits non-céréaliers appliquant le procédé non destructif de détermination de la maturité et de la qualité d'un fruit non-céréalier tel que décrit ci-dessus auquel les étapes suivantes auront été ajoutées :
- amener individuellement chaque fruit non-céréaliers à au moins une zone d'exposition,
- séparer lesdits fruits en différentes classes en fonction de leur signaux individuels.

Ce procédé est mis en pratique par l'appareil de tri selon l'invention généralement décrit ci-dessus et plus en détail ci-dessous.

### • Appareil de tri de fruits non-céréaliers :

L'appareil de tri de fruits non-céréaliers selon la présente invention comprend en particulier un dispositif d'alimentation de fruits non-céréaliers.

Les fruits non-céréaliers, telles que des baies de raisin, sont mises en défilement par le moyen d'un convoyeur, la vitesse de défilement du convoyeur éjecte les baies à son extrémité selon une trajectoire connue.

De manière préférée, l'appareil de tri de fruits non-céréaliers selon la présente invention peut être caractérisé en ce que ledit appareil est configuré pour trier des fruits comme décrits ci-dessus dans le procédé selon l'invention, par exemple choisis parmi des tomates, des raisins, des fraises, des framboises, des groseilles, des cerises, des mûres, des airelles rouges, des cassis, des baies d'aronia, des canneberges, des sureaux noirs et des myrtilles, préférentiellement des raisins tels que des raisins rouges.

L'appareil de tri de fruits non-céréaliers selon la présente invention comprend en particulier au moins un système de rayonnement configuré pour générer un ou plusieurs rayonnements électromagnétiques.

Dans un mode de réalisation particulier, l'appareil de tri de fruits non-céréaliers selon la présente invention comprend deux systèmes de rayonnements configurés pour émettre au moins deux rayonnements électromagnétiques.

L'appareil de tri de fruits non-céréaliers selon la présente invention comprend en outre au moins une zone de détection configurée pour mesurer et optionnellement analyser les signaux retours issus du ou des systèmes de rayonnements. Une zone de détection et deux systèmes de rayonnements configurés pour que les signaux retours atteignent ladite zone de détection, peuvent constitués un ensemble d'analyse de maturité de fruit non-céréaliers. L'appareil de tri de fruits non-céréaliers selon la présente invention peut comprendre plusieurs ensembles d'analyse de maturité de fruits non-céréaliers chacun disposé pour exposer lesdits fruits non-céréaliers selon des angles différents.

Dans un mode de réalisation particulier, la distance de la zone de détection vis-à-vis de fruits irradiés est adaptée pour avoir à la fois la plus grosse zone de captation des signaux retours, sans que le moyen de détection ne subissent des aléas liés à sa proximité avec les fruits (pulvérisation de jus, de saletés ou autre).

En outre, chaque ensemble d'analyse peut comprendre un dispositif d'analyse des signaux reçus, tel qu'une carte électronique ou un processeur électronique, ou un dispositif tel qu'un ordinateur.

L'appareil de tri de fruits non-céréaliers selon la présente invention comprend en particulier un système de séparation desdits fruits qui intervient en fonction des signaux retours, qui peut prendre la forme d'un moyen de fourniture d'air comprimé ou encore d'un bras articulé permettant l'expulsion desdits fruits indésirables.

Ainsi, l'appareil de tri selon la présente invention met en œuvre le procédé non destructif de détermination de la maturité et de la qualité d'un fruit non-céréalier selon la présente invention.

En outre, l'appareil de tri selon la présente invention est adapté à la taille des fruits triés. La taille de ces fruits peut être, par exemple dans le cas du raisin, de l'ordre de 10 à 20 mm de diamètre, préférentiellement de 11 à 15 mm de diamètre.

### • Appareil d'analyse, en particulier portatif :

Dans un mode de réalisation particulier, l'objet de la présente invention peut être appliqué à un appareil d'analyse, en particulier portatif, de végétaux sur le terrain. Par exemple, de manière avantageuse, le procédé de suivi de l'état ou de l'évolution d'une culture selon la présente invention peut être appliquée à un dispositif portatif selon FR2916850A1 ou d'un type équivalent.

En effet, le dispositif décrit dans FR2916850A1, qui par sa construction et son mode d'utilisation, est mis en contact sur la plante, et en particulier sur les fruits, garantit d'avoir toujours la même distance excitation-cible. L'appareil selon la présente invention peut donc reprendre les mêmes caractéristiques que celles décrites dans FR2916850A1, adapté pour exposer le/les fruit(s) analysé aux deux longueurs d'ondes dans les conditions telles que décrites ci-dessus.

De manière particulière, un tel appareil (portatif) comprend un boîtier portable autoali-menté présentant une première face portant des moyens d'interface utilisateur et une deuxième face dite de mesure de mesure dirigée vers une direction de mesure, cette face de mesure comportant d'une part sur sa périphérie une surface portant les moyens d'excitation et d'autre part en son centre une partie s'étendant dans la direction de mesure, renfermant au moins une partie de l'électronique de détection et portant les moyens de détection sur sa face du côté de la direction de mesure.

Dans un mode de réalisation particulier, l'appareil (portatif) selon la présente invention comprend un dispositif de positionnement dans l'espace, tel qu'un GPS, ce qui permet par exemple de faire un lien précis ente les mesures effectuées et une culture ou partie de culture.

En outre, dans le contexte selon la présente invention, les systèmes de rayonnement d'un appareil (portatif) selon la présente invention comprennent une ou plusieurs sources à base de diodes électroluminescentes (LED). L'avantage des LED est une limitation de la consommation d'énergie.

Dans un mode de réalisation particulier, un tel appareil comprend au moins un émetteur d'excitation fixé sur un élément radiateur dont la forme détermine la position et l'orientation dudit émetteur ou détecteur.

En particulier, l'appareil portatif comprend des moyens de gestion agencés pour fournir au moins une mesure de fluorescence par exemple un rayonnement à 650nm - dite « d'excitation », et au moins une excitation de détection (par exemple à 750 nm, utilisé en réflexion) déterminées pour être combinées et corrélées entre elles, comme décrit présentement.

De manière préférée, les moyens de gestion (et de traitement) sont agencés pour :
- commander le(s) système(s) de rayonnement par impulsions,
- détecter les pics de fluorescence générée par ces impulsions grâce à des détecteurs, pouvant être amplifiés pour réaliser la réjection de la lumière ambiante par une boucle de contre-réaction, et
- traiter le signal de fluorescence détecté et fournir la mesure de fluorescence.

De manière préférée, les moyens de gestion (et de traitement) sont agencés pour :
- commander le(s) système(s) de rayonnement par impulsions,
- détecter les pics de réflexion générée par ces impulsions grâce à des détecteurs, pouvant être amplifiés pour réaliser la réjection de la lumière ambiante par une boucle de contre-réaction, et
- traiter le signal de réflexion détecté.

De manière préférée, les moyens de gestion et de traitement sont agencés pour :
- commander le(s) système(s) de rayonnement selon une fréquence incluant une modulation, et
- traiter le signal de détection de fluorescence, par exemple en démodulation de phase de façon à obtenir la rejection de la lumière ambiante, et
- fournir la mesure de fluorescence.

De manière préférée, les moyens de gestion et de traitement sont agencés pour :
- commander le(s) système(s) de rayonnement selon une fréquence incluant une modulation,
- traiter le signal de détection de réflexion, par exemple en démodulation de phase de façon à obtenir la rejection de la lumière ambiante, et
- fournir la mesure de réflexion.

Ainsi, l'appareil (en particulier portatif) selon la présente invention, permet la mise en œuvre d'un procédé configuré pour le suivi de l'évolution d'une culture dans le temps, caractérisé en ce qu'il permet de comparer une pluralité de mesures réalisées au cours du temps pour un composé déterminé dans un même végétal ou sur une même parcelle.

De manière préférée, le procédé est mis en œuvre pour évaluer la maturation ou la qualité ou la composition de fruits ou légumes par mesure de la teneur d'au moins un marqueur de maturité, tel qu'un anthocyane.

De manière préférée, le procédé est mis en œuvre pour qu'au moins une mesure d'une pluralité de végétaux est réalisée à la volée, au fur et à mesure d'un déplacement de l'appareil de mesure (en particulier portatif) selon la présente invention.

### FIGURES

On décrira ci-après, à titre d'exemples non limitatifs, des formes d'exécution de la présente invention, en référence aux figures annexées sur lesquelles :
[Fig.1] est une représentation schématique du procédé de tri selon la présente invention appliquée sur une chaine de tri de fruits non-céréaliers ;
[Fig.2] représente un diagramme des phases pour l'exposition de raisins.

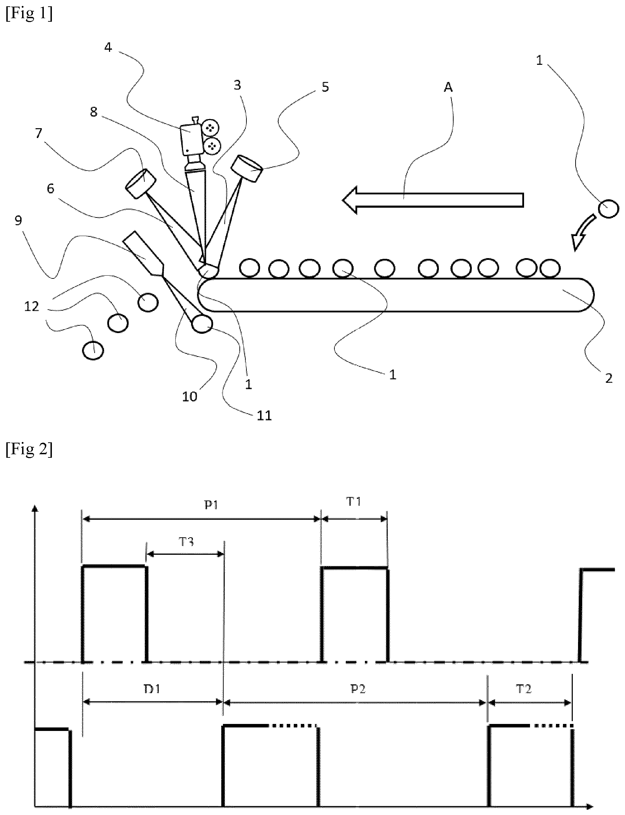

En référence à la figure 1, des raisins 1 sont mis en défilement (défilement représenté par la flèche A) par le moyen d'un convoyeur 2, la vitesse de défilement du convoyeur peut éjecter les baies à son extrémité selon une trajectoire connue, ou le raisin 1 peut être analysé sur le convoyeur 2 (non représenté ici).

Une première onde électromagnétique 3 est focalisée sur les baies à l'endroit de leur passage devant la caméra 4. La première onde électromagnétique est produite par un premier émetteur 5. Une deuxième onde électromagnétique 6 est focalisée sur les baies à l'endroit de leur passage devant la caméra 4. La deuxième onde électromagnétique est produite par un deuxième émetteur 7. Les signaux-retours 8 sont ainsi captés par la caméra 4. Ainsi, pour répondre à la mise en œuvre du procédé de mesure de fluorescence émise (750 nm) et de réflexion (750 nm) les émetteurs 5,7 sont allumés séquentiellement pour que la caméra 4 mesure successivement la fluorescence due à l'excitation de chaque éclairage et le signal de réflexion.

Ainsi, la caméra 4 peut être dans le cas particulier représenté une caméra monochrome infrarouge pourvue d'un filtre passe bande à 750 nm.

L'objectif de la caméra 4 peut être équipé d'un filtre passe bande pour la spécialiser sur la longueur d'onde de la fluorescence recherchée

L'axe de vision de la caméra 4 peut être perpendiculaire au plan du convoyeur 2 qui met le raisin 1 en défilement (représenté par la flèche A).

Le capteur de la caméra 4 peut être orienté dans sa longueur de manière à être transversal à l'axe de défilement du convoyeur 2.

L'objectif de la caméra 4 peut permettre le réglage de la netteté de la caméra 4 à la distance focale.

L'un des émetteurs 5,7 peut être filtré passe bas pour couper les ondes électromagnétiques spécifiques à la fluorescence et ainsi être totalement dédié à fournir un signal-retour 8 de réflexion.

Les émetteurs 5,7 peuvent être dimensionnés pour éclairer toutes les baies 1 mises en défilement sur la largeur du convoyeur 2. La caméra 4 peut être équipée d'un objectif de manière à recevoir des signaux-retours 8 sur toute la largeur du convoyeur 2.

La focale et l'intensité de l'exposition sont dimensionnées pour positionner les émetteurs 5,7 à bonne distance du raisin.

Les émetteurs 5,7 peuvent comprendre des LEDS pour fournir les ondes électromagnétiques voulues. Ces LEDS peuvent être disposées de manière à obtenir une exposition sur une ligne continue sur une seule face des fruits exposés.

Le ou les expositions sont réglables en orientation pour les faire converger en un seul endroit sur la trajectoire connue du fruit non céréalier.

Les émetteurs 5,7 et la caméra 4 peuvent être suffisamment protégés du rayonnement de l'environnement extérieur pour ne pas affecter les mesures.

Les émetteurs 5,7 peuvent être configurés de manière éclairer au minimum la largeur du convoyeur 2 qui transporte les fruits à trier.

Les émetteurs 5,7 peuvent être au-dessus du convoyeur 2 ou peu après la sortie du convoyeur 2.

Les émetteurs 5,7 peuvent être décalés pour ne pas se trouver dans le champ de la caméra 4.

Un moyen d'éjection 9, telle qu'une buse, permet de retirer les fruits ou les objets non désirés. C'est en effet un avantage optionnel de la présente invention que de pouvoir identifier des objets non voulus si on le souhaite en comparant l'image obtenue suite à l'exposition du fruit passant devant la caméra 4 et le contour général attendu du fruit à trier. D'autres systèmes de tri basés par exemple sur la couleur ou la forme peuvent en outre être ajoutés pour augmenter la précision du tri.

Ici, c'est un jet d'air 10 qui permet l'éjection du fruit non désiré 11.

Il y a autant de moyens d'éjection 9 que nécessaire pour que le tri puisse être opérationnel sur la largeur du convoyeur.

Ainsi, les fruits triés 12 que l'on souhaite garder sont isolés et récupérés.

Un système de gestion est mis en œuvre pour gérer le séquençage de l'éclairage et la synchronisation de la mesure de fluorescence. Ainsi, en figure 2 est représenté un diagramme de phases particulièrement bien adapté à la chaine de tri selon la figure 1 dans laquelle :

P1 représente une période excitation N°1. P1 peut permettre de définir la résolution du système ;
P2 représente une période excitation N°2 ;
T1 représente une durée excitation N°1 ;
T2 représente une durée excitation N°2, la plus sensible (absorbée), pourra être ajustée pour garantir suffisamment de signal ;
T3 représente un temps disponible pour la mesure du bruit ; et
D1 représente un décalage de phase pour assurer la synchronisation de la référence et de la mesure.

La durée d'excitation de la longueur d'onde à 625 nm est comprise entre 100 et 300 µS, de préférence 240 µS. Le temps de cycle total est compris entre 300 et 600 µS, de préférence 500 µS.

Le séquençage des excitations doit pouvoir permettre la mesure en plusieurs endroits d'un même fruit à trier (ici du raisin) qui est en défilement à une vitesse connue. Le nombre de mesures effectuées sur un fruit à trier de taille moyenne définit la résolution du système. Cette résolution est en adéquation avec le niveau de précision recherché et le débit de fruit souhaité.

La durée de l'exposition dans les longueurs d'ondes et leurs périodes, peuvent être gérés par un automate adapté pour la vision industrielle.

Le décalage de phases entre plusieurs longueurs d'ondes d'exposition peut d'ailleurs être géré par l'automate.

Le résultat du traitement est rendu sous une forme simple interprétable par l'opérateur de manière à lui permettre le réglage de la sélection à réaliser.

Une juxtaposition des images issus de fluorescence émise et des images de la réflexion permet de repérer et quantifier les baies à faible niveau de chlorophylle.

Le dispositif peut prévoir pour les éléments de faible niveau de chlorophylle de réaliser une analyse d'images afin de discriminer les baies des autres éléments de la récoltes.

Il est avantageux alors que le système de gestion pilote les moyens de tri de manière à réaliser la sélection réglée par l'opérateur. Ce réglage est modifiable à tout moment en cours de fonctionnement.

L'opérateur pourra déterminer auparavant via une analyse appropriée un seuil de sélection, soit par la réalisation au préalable d'un échantillon analysé par d'autres moyens, soit par appréciation en marche de la répartition quantitative entre plusieurs lots constitués et par un ajustement du seuil de sélection.

### EXEMPLES

A titre d'illustration, des mesures d'absorbances selon la présente invention faites en spectrophotométrie à 520 (c'est-à-dire une longueur d'onde cohérente avec les teneurs en anthocyanes) sur des raisins ont été réalisées.

Ainsi, des écarts entre des lots plus mûrs et moins mûrs ont pu être mesurés avec le procédé selon la présente invention :

**Tableau 1 : résultats obtenus en 2020**

| Cépage du raisin | Ecart en pourcentage entre les fruits les plus mûrs par rapport aux moins mûrs |
|---|---|
| Merlot | +25,5% |
| Cabernet 1 | +13,6% |
| Cabernet 2 | +12,6% |

N.B. : le procédé de 2020 différait de celui mis en œuvre en 2021 par le fait que le classement des fruits était fait manuellement selon les résultats donnés par le procédé. Le passage des fruits était fait manuellement un à un dans le système où il était excité selon la longueur d'onde adéquate, l'ordre de passage conservé pour chaque fruit, le procédé était appliqué manuellement à chaque fruit lors du visionnage des images enregistrées de sa Fluorescence. La Fluorescence due à chaque excitation était enregistrée séparément.

**Tableau 2 : résultats obtenus en 2021**

| Cépage du raisin | Ecart en pourcentage entre les fruits les plus mûrs par rapport aux moins mûrs |
|---|---|
| Merlot 1 | +48% |
| Merlot 2 | +37% |
| Cabernet 1 | +58% |

Le procédé de 2021 a permis de montrer un niveau de maturité global meilleur sur les raisins testés en comparaison avec ceux de 2020.

Le banc de mesure de 2021 était entièrement automatisé jusqu'à la classification physique des fruits selon le procédé.

Les analyses de teneur en Anthocyanes sur vin fini à partir des raisins ainsi classés pendant les essais 2021 montrent les mêmes tendances

## Revendications

1. Procédé non destructif de détermination de la maturité et de la qualité d'un fruit non-céréalier comprenant au moins deux étapes d'expositions dudit fruit à au moins deux rayonnements électromagnétiques différents et de mesures de leurs signaux retours, dans lequel:
- l'une des étapes d'expositions dudit fruit comprend la fourniture d'au moins une longueur d'onde susceptible de provoquer un signal de fluorescence de chlorophylle et une mesure, le cas échéant, dudit signal de fluorescence de chlorophylle ; et
- l'une des étapes d'exposition dudit fruit à au moins un rayonnement électromagnétique comprend la fourniture d'au moins une longueur d'onde choisie, susceptible de générer un signal de réflexion sur ledit fruit, et une mesure, le cas échéant, dudit signal de réflexion,
**caractérisé en ce que** ladite au moins une longueur d'onde susceptible de provoquer une fluorescence de la chlorophylle est comprise entre 590 nm et 660 nm, et **en ce que**
les mesures du signal de fluorescence de la chlorophylle et du signal de réflexion sont effectuées par un même capteur.

2. Procédé selon la revendication 1, **caractérisé en ce que** le capteur est une caméra optionnellement pourvue d'un filtre passe-bande, par exemple un filtre passe-bande calibré à 750 nm.

3. Procédé selon l'une quelconque des revendications 1 ou 2, **caractérisé en ce que** d'une part, la fourniture d'au moins une longueur d'onde susceptible de provoquer un signal de fluorescence de chlorophylle, et d'autre part la fourniture d'au moins une longueur d'onde choisie susceptible de générer un signal de réflexion sur ledit fruit, sont séquencés et/ou synchronisés, avec optionnellement au moins un temps de pause pour permettre la mesure du bruit de fond.

4. Procédé selon l'une quelconque des revendications 1 à 3, **caractérisé en ce que** ladite au moins une longueur d'onde choisie pour générer une réflexion sur ledit fruit est choisie avec une longueur d'onde significativement supérieure à la longueur d'onde d'excitation de la chlorophylle.

5. Procédé selon la revendication 3 **caractérisé en ce que** la longueur d'onde significativement supérieure à la longueur d'onde d'excitation de la chlorophylle est comprise entre 700 nm et 900 nm, préférentiellement de 750 nm plus ou moins 10 nm, et **en ce que** ladite au moins une longueur d'onde susceptible de provoquer une fluorescence de la chlorophylle est de 625 nm plus ou moins 10 nm.

6. Procédé selon l'une quelconque des revendications 1 à 5 **caractérisé en ce que** le fruit non-céréalier contient au moins un pigment choisi parmi le lycopène, le bêtacarotène, l'anthocyane et la lutéine, préférentiellement l'anthocyane et/ou le lycopène

7. Procédé selon l'une quelconque des revendications 1 à 6 **caractérisé en ce que** le fruit non-céréalier est choisi parmi une tomate, un raisin, une fraise, une framboise, une groseille, une cerise, une mûre, une airelle rouge, un cassis, une baie d'aronia, une canneberge, un sureau noir et une myrtille, préférentiellement un raisin.

8. Appareil de tri de fruits non-céréaliers comprenant :
- un dispositif d'alimentation de fruits non-céréaliers,
- optionnellement au moins un moyen d'écarter des objets qui ne sont pas des fruits ;
- au moins un système de rayonnement configuré pour générer au moins un rayonnement électromagnétique ;
- au moins une zone de détection configurée pour mesurer et optionnellement analyser les signaux retours issus dudit au moins un système de rayonnement ;
- un système de séparation desdits fruits activable en fonction des signaux retours,
**caractérisé en ce que** :
- ledit au moins un système de rayonnement est configuré de manière à exposer lesdits fruits à au moins deux rayonnements électromagnétiques différents ;
- l'un desdits au moins deux rayonnements électromagnétiques comprend au moins une longueur d'onde susceptible de provoquer une fluorescence de chlorophylle ;
- l'un desdits au moins deux rayonnements électromagnétiques comprend au moins une longueur d'onde choisie pour générer une réflexion sur ledit fruit, telle que 750 nm ;
- ledit au moins un système de rayonnements et la zone de détection sont disposés de manière à :
- ce qu'au moins deux signaux retours générés par ledit au moins un système de rayonnement sont captés sur une même zone de détection, ou
- être d'un même côté des fruits
**caractérisé en ce que** ladite au moins une longueur d'onde susceptible de provoquer une fluorescence de la chlorophylle est comprise entre 590 nm et 660 nm,
et **en ce que** les mesures du signal de fluorescence de la chlorophylle et du signal de réflexion sont effectuées par un même capteur.

9. Un procédé de tri de fruits non-céréaliers comprenant les étapes suivantes :
- amener individuellement chaque fruit non-céréaliers à au moins une zone d'exposition,
- exposer ledit fruit dans ladite au moins une zone d'exposition à au moins deux rayonnements électromagnétiques différents,
- réceptionner et analyser les signaux retours de l'étape d'exposition,
- séparer lesdits fruits en différentes classes en fonction de leur signaux individuels,
dans lequel :
- l'un des au moins deux rayonnements électromagnétiques différents comprend au moins une longueur d'onde susceptible de provoquer une fluorescence de chlorophylle comme signal retour ;
- l'un des au moins deux rayonnements électromagnétiques différents comprend au moins une longueur d'onde choisie pour générer une réflexion sur ledit fruit comme signal retour ; et
- les valeurs qui définissent les classes sont préétablies ou choisies en fonction d'une répartition des signaux de réflexion et de fluorescence de chlorophylle émis par un échantillon de fruits non-céréaliers possédant des propriétés connues **caractérisé en ce que** ladite au moins une longueur d'onde susceptible de provoquer une fluorescence de la chlorophylle est comprise entre 590 nm et 660 nm,
et **en ce que** les mesures du signal de fluorescence de la chlorophylle et du signal de réflexion sont effectuées par un même capteur.

## Patentansprüche

1. Zerstörungsfreies Verfahren zur Bestimmung der Reife und Qualität einer Nicht-Getreidefrucht, umfassend mindestens zwei Schritte des Aussetzens der Frucht gegenüber mindestens zwei verschiedenen elektromagnetischen Strahlungen und des Messens ihrer Rücksignale, wobei:
- einer der Schritte des Aussetzens der Frucht das Bereitstellen mindestens einer Wellenlänge, die in der Lage ist, ein Chlorophyllfluoreszenzsignal zu verursachen, und gegebenenfalls eine Messung des Chlorophyllfluoreszenzsignals beinhaltet; und
- einer der Schritte des Aussetzens der Frucht gegenüber mindestens einer elektromagnetischen Strahlung das Bereitstellen mindestens einer ausgewählten Wellenlänge, die in der Lage ist, ein Reflexionssignal an der Frucht zu erzeugen, und gegebenenfalls eine Messung des Reflexionssignals umfasst,
**dadurch gekennzeichnet, dass** die mindestens eine Wellenlänge, die in der Lage ist, Chlorophyllfluoreszenz zu verursachen, zwischen 590 nm und 660 nm liegt, und dass
die Messungen des Chlorophyllfluoreszenzsignals und des Reflexionssignals vom gleichen Sensor durchgeführt werden.

2. Verfahren nach Anspruch 1, **dadurch gekennzeichnet, dass** der Sensor eine Kamera ist, die optional mit einem Bandpassfilter, beispielsweise mit einem auf 750 nm kalibrierten Bandpassfilter versehen ist.

3. Verfahren nach einem der Ansprüche 1 oder 2, **dadurch gekennzeichnet, dass** zum einen das Bereitstellen mindestens einer Wellenlänge, die in der Lage ist, ein Chlorophyllfluoreszenzsignal zu verursachen, und zum anderen das Bereitstellen mindestens einer ausgewählten Wellenlänge, die ein Reflexionssignal an der Frucht erzeugen kann, sequenziert und/oder synchronisiert werden, gegebenenfalls mit mindestens einer Pausenzeit, um die Messung des Hintergrundrauschens zu ermöglichen.

4. Verfahren nach einem der Ansprüche 1 bis 3, **dadurch gekennzeichnet, dass** die mindestens eine Wellenlänge, die zur Erzeugung von Reflexion an der Frucht ausgewählt wird, mit einer Wellenlänge ausgewählt wird, die wesentlich größer als die Anregungswellenlänge von Chlorophyll ist.

5. Verfahren nach Anspruch 3, **dadurch gekennzeichnet, dass** die Wellenlänge, die deutlich größer als die Anregungswellenlänge des Chlorophylls ist, zwischen 700 nm und 900 nm, vorzugsweise 750 nm plus oder minus 10 nm, liegt und dass die mindestens eine Wellenlänge, die in der Lage ist, Chlorophyllfluoreszenz zu verursachen, 625 nm plus oder minus 10 nm beträgt.

6. Verfahren nach einem der Ansprüche 1 bis 5, **dadurch gekennzeichnet, dass** die Nicht-Getreidefrucht mindestens ein Pigment, ausgewählt aus Lycopin, Beta-Carotin, Anthocyan und Lutein, vorzugsweise Anthocyan und/oder Lycopin, enthält.

7. Verfahren nach einem der Ansprüche 1 bis 6, **dadurch gekennzeichnet, dass** die Nicht-Getreidefrucht aus einer Tomate, Weinbeere, Erdbeere, Himbeere, Johannisbeere, Kirsche, Brombeere, Preiselbeere, schwarzen Johannisbeere, Aroniabeere, Moosbeere, Holunderbeere und Blaubeere, vorzugsweise einer Weinbeere, ausgewählt ist.

8. Einrichtung zum Sortieren von Nicht-Getreidefrüchten, die Folgendes umfasst:
- eine Vorrichtung zur Zufuhr von Nicht-Getreidefrüchten,
- gegebenenfalls mindestens ein Mittel zum Verwerfen von Objekten, die nicht Früchte sind;
- mindestens ein Strahlungssystem, das dazu ausgelegt ist, mindestens eine elektromagnetische Strahlung zu erzeugen;
- mindestens eine Detektionszone, die dazu ausgelegt ist, die Rücksignale, die von dem mindestens einen Strahlungssystem stammen, zu messen und optional zu analysieren;
- ein System zur Trennung der genannten Früchte, das entsprechend den Rücksignalen aktiviert werden kann;
**dadurch gekennzeichnet, dass**:
- das mindestens eine Strahlungssystem dazu ausgelegt ist, die Früchte mindestens zwei verschiedenen elektromagnetischen Strahlungen auszusetzen;
- eine der mindestens zwei elektromagnetischen Strahlungen mindestens eine Wellenlänge umfasst, die in der Lage ist, Chlorophyllfluoreszenz zu verursachen;
- eine der mindestens zwei elektromagnetischen Strahlungen mindestens eine Wellenlänge umfasst, die ausgewählt ist, um eine Reflexion an der Frucht zu erzeugen, wie etwa 750 nm;
- das mindestens eine Strahlungssystem und die Detektionszone so angeordnet sind, dass:
- mindestens zwei von dem mindestens einen Strahlungssystem erzeugte Rücksignale auf einer gleichen Detektionszone aufgenommen werden, oder
- sie auf einer gleichen Seite der Früchte liegen,
**dadurch gekennzeichnet, dass** die mindestens eine Wellenlänge, die in der Lage ist, Chlorophyllfluoreszenz zu verursachen, zwischen 590 nm und 660 nm liegt,
und dass die Messungen des Chlorophyllfluoreszenzsignals und des Reflexionssignals vom gleichen Sensor durchgeführt werden.

9. Verfahren zum Sortieren von Nicht-Getreidefrüchten, umfassend die folgenden Schritte:
- Bringen jeder Nicht-Getreidefrucht einzeln in mindestens eine Aussetzungszone;
- Aussetzen der Frucht in der mindestens einen Aussetzungszone gegenüber mindestens zwei verschiedenen elektromagnetischen Strahlungen;
- Empfangen und Analysieren der Rücksignale des Aussetzungsschritts,
- Trennen der genannten Früchte nach ihren individuellen Signalen in verschiedene Klassen;
wobei:
- eine der mindestens zwei verschiedenen elektromagnetischen Strahlungen mindestens eine Wellenlänge umfasst, die in der Lage ist, Chlorophyllfluoreszenz als ein Rücksignal zu bewirken;
- eine der mindestens zwei verschiedenen elektromagnetischen Strahlungen mindestens eine Wellenlänge umfasst, die ausgewählt ist, um eine Reflexion auf der Frucht als ein Rücksignal zu erzeugen; und
- die Werte, die die Klassen definieren, gemäß einer Verteilung der Reflexions- und Chlorophyllfluoreszenzsignale, die von einer Probe von Nicht-Getreidefrüchten mit bekannten Eigenschaften emittiert werden, vorbestimmt oder ausgewählt werden, **dadurch gekennzeichnet, dass** die mindestens eine Wellenlänge, die in der Lage ist, eine Chlorophyllfluoreszenz zu verursachen, zwischen 590 nm und 660 nm liegt,
und dass die Messungen des Chlorophyllfluoreszenzsignals und des Reflexionssignals vom gleichen Sensor durchgeführt werden.

## Claims

1. Non-destructive method for determining the maturity and quality of a non-cereal fruit comprising at least two steps of exposing said fruit to at least two different electromagnetic emissions and of measuring their feedback signals, in which:
- one of the steps of exposing said fruit comprises delivery of at least one wavelength capable of provoking a chlorophyll-fluorescence signal and measurement, where appropriate, of said chlorophyll-fluorescence signal; and
- one of the steps of exposing said fruit to at least one electromagnetic emission comprises delivery of at least one selected wavelength capable of generating a reflection signal due to reflection from said fruit, and measurement, where appropriate, of said reflection signal,
**characterized in that** said at least one wavelength capable of provoking fluorescence of chlorophyll is between 590 nm and 660 nm, and **in that**
the measurements of the fluorescence signal of the chlorophyll and the reflection signal are taken by the same sensor.

2. Method according to Claim 1, **characterized in that** the sensor is a camera that is optionally provided with a band-pass filter, for example a band-pass filter calibrated at 750 nm.

3. Method according to either of Claims 1 and 2, **characterized in that**, on the one hand, the delivery of at least one wavelength capable of provoking a chlorophyll-fluorescence signal, and on the other hand, the delivery of at least one selected wavelength capable of generating a reflection signal due to reflection from said fruit, are sequenced and/or synchronized, with optionally at least one pause to allow measurement of background noise.

4. Method according to any of Claims 1 to 3, **characterized in that** said at least one wavelength selected to generate a reflection from said fruit is selected with a wavelength significantly longer than the wavelength of excitation of the chlorophyll.

5. Method according to Claim 3, **characterized in that** the wavelength significantly longer than the wavelength of excitation of the chlorophyll is between 700 nm and 900 nm, and preferably 750 nm plus or minus 10 nm, and **in that** said at least one wavelength capable of provoking fluorescence of chlorophyll is 625 nm plus or minus 10 nm.

6. Method according to any of Claims 1 to 5, **characterized in that** the non-cereal fruit contains at least one pigment selected from lycopene, beta-carotene, anthocyanin and lutein, preferably anthocyanin and/or lycopene.

7. Method according to any of Claims 1 to 6, **characterized in that** the non-cereal fruit is selected from a tomato, grape, strawberry, raspberry, redcurrant, cherry, blackberry, lingonberry, blackcurrant, aronia berry, cranberry, elderberry and blueberry, but is preferably a grape.

8. Apparatus for sorting non-cereal fruits comprising:
- a device for supplying non-cereal fruits,
- optionally at least one means for discarding objects that are not fruits;
- at least one emitting system configured to generate at least one electromagnetic emission;
- at least one detection region configured to measure and optionally analyse the feedback signals generated by said at least one emitting system;
- a system for separating said fruits, which is activatable depending on the feedback signals,
**characterized in that**:
- said at least one emitting system is configured to expose said fruit to at least two different electromagnetic emissions;
- one of said at least two electromagnetic emissions comprises at least one wavelength capable of provoking fluorescence of chlorophyll;
- one of said at least two electromagnetic emissions comprises at least one wavelength selected so as to generate a reflection from said fruit, such as 750 nm;
- said at least one emitting system and the detection region are arranged in such a way:
- that at least two feedback signals generated by said at least one emitting system are picked up in the same detection region, or
- as to be on the same side of the fruits
**characterized in that** said at least one wavelength capable of provoking fluorescence of chlorophyll is between 590 nm and 660 nm,
and **in that** the measurements of the fluorescence signal of the chlorophyll and the reflection signal are taken by the same sensor.

9. Method for sorting non-cereal fruits comprising the following steps:
- bringing each non-cereal fruit individually into at least one exposure region,
- exposing said fruit in said at least one exposure region to at least two different electromagnetic emissions,
- receiving and analysing the feedback signals of the exposing step,
- separating said fruits into different classes depending on their individual signals,
in which:
- one of the at least two different electromagnetic emissions comprises at least one wavelength capable of provoking fluorescence of chlorophyll by way of feedback signal;
- one of the at least two different electromagnetic emissions comprises at least one wavelength selected so as to generate a reflection from said fruit by way of feedback signal; and
- the values that define the classes are preset or selected depending on a distribution of the chlorophyll-fluorescence and reflection signals emitted by a sample of non-cereal fruits possessing known properties **characterized in that** said at least one wavelength capable of provoking fluorescence of chlorophyll is between 590 nm and 660 nm,
and **in that** the measurements of the fluorescence signal of the chlorophyll and the reflection signal are taken by the same sensor.
